(19) Europäisches Patentamt
European Patent Office
Office européen des brevets

(11) Veröffentlichungsnummer: **0 166 986**
**A1**

# (12) EUROPÄISCHE PATENTANMELDUNG

(21) Anmeldenummer: 85106846.0

(22) Anmeldetag: 03.06.85

(51) Int. Cl.[4]: C 07 D 273/08
//A61K31/315, C07C113/04

(30) Priorität: 06.06.84 DE 3421062

(43) Veröffentlichungstag der Anmeldung:
08.01.86 Patentblatt 86/2

(84) Benannte Vertragsstaaten:
BE CH DE FR GB IT LI NL

(71) Anmelder: Deutsches Krebsforschungszentrum
Im Neuenheimer Feld 280
D-6900 Heidelberg 1(DE)

(72) Erfinder: Kolar, George F., Dr. Dipl.-Chem.
Banngartenstrasse 19a
D-6900 Heidelberg(DE)

(72) Erfinder: Schendzielorz, Michael, Dipl.-Chem.
Siebseeweg 3a
D-6800 Mannheim(DE)

(74) Vertreter: Schön, Alfred, Dr.
Patentanwälte Müller-Boré, Deufel, Schön, Hertel
Lewald, Otto Isartorplatz 6 Postfach 26 02 47
D-8000 München 26(DE)

**(54) Disubstituierte 3,7-Diaryl-1,5,3,7-dioxadiazocine und Verfahren zu ihrer Herstellung.**

(57) Die Erfindung betrifft neue disubstituierte 3,7-Diaryl-1,5,3,7-dioxodiazocine der allgemeinen Formel

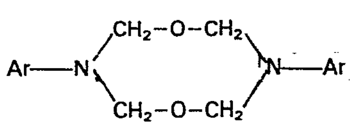

worin Ar eine durch einen elektronenziehenden Rest substituierte Arylgruppe ist. Diese Verbindungen sind Chemotherapeutika, insbesondere Immunsystemstimulantien.

EP 0 166 986 A1

# Disubstituierte 3,7-Diaryl-1,5,3,7-dioxadiazocine und Verfahren zu ihrer Herstellung

Es sind eine Reihe von N, N'-disubstituierten Dioxadiazocinen bekannt, die gemäß der jetzigen Nomenklatur als 3,7-subst.-1,5,3,7-Dioxadiazocine zu bezeichnen sind. Verwiesen sei beispielsweise auf die DE-OS 19 53 249 sowie Chemical Abstract 77 (1972) S. 512, 62041n sowie Chemical Abstracts 77 (1972) S. 512, 62042p.

Die Herstellung erfolgt z.B. nach der erstgenannten Literaturstelle durch Kondensation der Methylolverbindung $RCON(CH_2OH)_2$ bei 100°C in Gegenwart von p-$MeC_6H_4SO_3H$ oder durch Umsetzung von $H_2NCOR$ mit Paraformaldehyd in PhMe oder MeOH in Gegenwart von p-$MeC_6H_4SO_3H$. Diese Verbindungen werden als knitterfestmachende Verbindungen für zellulosehaltige Textilstoffe eingesetzt.

Die in Chemical Abstract 77 (1972) S. 512, 62041n gezeigten ähnlichen Verbindungen werden durch Behandlung von Octadecylcarbamat mit Paraformaldehyd in Xylol, das Toluolsulfonsäure enthält, bei 60°C für 3 h in entsprechender Weise hergestellt.

Die in Chemical Abstract 77 (1972) S. 512, 62042 p gezeigten 2,8-Dichlor-5,11-dimethyl-6,12-epoxy-5,6,11-12-tetrahydro-6,12-diphenyldibenzo[b,f] [1,5] diazocine werden durch Dehydrieren von 2,5(MeNH)$ClC_6H_3$COPh mit Natriumhydrid in Benzol/DMF erhalten. Es wird angegeben, daß die Verbindung als Arzneimittel für das zentrale Nervensystem anwendbar ist.

Es wurde nun eine neue Gruppe von Dioxodiazocinen gefunden, die der allgemeinen Formel

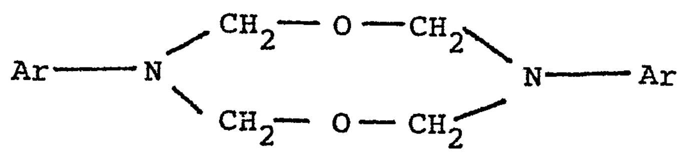

entsprechen, worin Ar eine durch einen elektronenziehenden Rest substituierte Arylgruppe ist.

Als Arylgruppe wird die Phenylgruppe bevorzugt, sehr geeignet ist jedoch auch die Pyridylgruppe, die gegebenenfalls auch nichtsubstituiert sein kann.

Hervorzuheben sind die Verbindungen, in denen Ar für eine Phenylgruppe steht, die vorzugsweise in p-Stellung substituiert ist.

Die elektronenziehenden Gruppen werden vorzugsweise aus solchen Gruppen ausgewählt, die eine Hammet-Konstante $\sigma$ von 0,20 bis 0,80 entsprechende der Hammet-Gleichung log $(k/k_o) = \sigma\rho$ besitzen.

Insbesondere besteht die elektronenziehende Gruppe aus einer gegebenenfalls verzweigten halogenierten Niedrigalkylgruppe mit 1 bis 6 Kohlenstoffatomen, einer halogenierten Cycloalkylgruppe mit bis zu 8 Kohlenstoffatomen, dem Rest $COOR_1$, worin $R_1$ eine Niedrigalkylgruppe mit 1 bis 3 Kohlenstoffatomen bedeutet, oder aus der Cyanogruppe oder Nitrogruppe, wobei von diesen Gruppen die folgenden Gruppen besonders hervorzuheben sind: -CN, $-NO_2$, $-COOCH_3$, $-COOCH_2CH_3$ und $CF_3$.

Die Herstellung der erfindungsgemäßen disubstituierten 3,7-Diaryl-1,5,3,7-dioxadiozocine erfolgt nach einem neuen Verfahren, welches darin besteht, daß

mit einer elektronenziehenden Gruppe substituiertes Ar-$NH_2$, vorzugsweise p-substituiertes Anilin, in Chlorwasserstoffsäure, insbesondere in einer 37 %igen Chlorwasserstoffsäure, mit Nitrit in herkömmlicher Weise diazotiert wird, d.h. unterhalb 0°C, insbesondere zwischen 0 und -5°C, die erhaltene Diazoniumsalzlösung einer vorzugsweise auf -5 bis -10°C gekühlten überschüssigen Mischung aus Methylamin und Formaldehyd zugesetzt wird, wobei die Zugabe vorzugsweise tropfenweise erfolgt, und wobei das Verhältnis Methylamin/Formaldehyd derart gewählt wird, daß nach der Zugabe der Diazoniumsalzlösung der pH-Wert der Reaktionsmischung zwischen 4 und 5 liegt, anschließend der pH-Wert der Reaktionsmischung herabgesetzt wird, und zwar vorzugsweise auf 1 unter Verwendung von Chlorwasserstoffsäure, worauf die Reaktionsmischung stehengelassen wird, vorzugsweise über Nacht, und das ausgefallene Dioxodiazocin in üblicher Weise isoliert wird.

Die erfindungsgemäßen Dioxodiazocine eignen sich als Chemotherapeutika, insbesondere als Immunsystemstimulantien.

Der achtgliedrige Ring der erfindungsgemäßen Dioxodiazocine wurden nicht nur durch $^1H$ und $^{13}C$ Kernresonanz (NMR) und Massenspektrometrie, sondern auch durch die Röntgenstruktur gesichert.

Biologische Prüfung (Ames-Test) zeigen die biologische Wirksamkeit.

Das folgende Beispiel erläutert die Erfindung.

Herstellung von para-Trifluormethyl-3,7-diphenyl-1,5,3,7-dioxadiazocin

a) Herstellung der Diazoniumlösung von 4-Trifluormethylanilin. 0,025 Mol des Anilins werden in konzentrierter Salzsäure (37 %, 10 ml) gelöst und mit Wasser (20 ml) verdünnt. Die saure Lösung wird auf -10°C gekühlt und durch Zugabe von $NaNO_2$ (0,04 mol) in Wasser (10 ml) diazotiert. Nach vollständiger Diazotierung (Kontrolle durch Dünnschichtchromatographie) wird das überschüssige Nitrit mit Harnstoff zerstört und die Lösung mit Aktivkohle geklärt und filtriert. Die Temperatur der Diazoniumlösung soll 0°C nicht überschreiten.

b) Umsetzung zur gesuchten Verbindung
Eine wässrige Lösung von Formaldehyd (37%, 40 ml) und Methylamin (40%, 9 ml) werden bei Raumtemperatur gründlich durchgemischt und anschließend auf -10°C gekühlt. Die gekühlte Diazoniumlösung wird innerhalb von 20 Minuten tropfenweise zu der vorgemischten Methylamin/Formaldehyd-Lösung zugegeben. Nach Beendigung der Zugabe beträgt der pH-Wert des Gemisches 4-5. Durch Zugabe von konzentrierter Salzsäure (4 ml) wird der pH-Wert des Reaktionsgemisches auf pH 1 eingestellt. Dann wird noch eine halbe Stunde nachgerührt und das Reaktionsgemisch 12 Stunden im Kühlschrank (bei 4°C) stehen gelassen. Danach wird der Niederschlag abgesaugt, mit Wasser neutral gewaschen und über Kieselgel getrocknet. Das erhaltene Produkt wird schließlich aus Benzol umkristallisiert. Die Ausbeute beträgt 40 bis 60% der Theorie. F.= 194°C

Beispiele 3 bis 5

Nach der gleichen Arbeitsweise wurden analoge Dioxodiazocine mit folgenden 4-Substituenten in vergleichbaren Ausbeuten synthetisiert:

| | | |
|---|---|---|
| $-CN$ | Ausbeute | F. = 227°C |
| $-NO_2$ | " | F. = 258°C |
| $-COOCH_3$ | " | F. = 217°C |
| $-COOCH_2CH_3$ | " | F. = 182°C |

1 Biologische Wertung:

a) Mutagenitätsprüfungen

Die Versuchsverbindung, gelöst in 40 µl an Dimethylsulphoxid und 100 µl eines Übernacht-Kulturmediums wurde zu 500 µl eines S9-Gemisches gegeben das 100 µl Leber S9 von mit Aroclor behandelten männlichen BD VI Ratten, 50 µl 0,15 M KCl - 5 mM Sørensen Puffer pH 7,4 , 50 µmol Sørensen Phosphat Puffer (pH 7,4), 2 µmol $NADP^+$, 2,5 µmol Glukose-6-phosphat und 4 µmol $MgCl_2$ enthält. Das Gemisch wurde bei 37°C 30 Minuten lang unter Schütteln vorinkubiert und dann mit 2 ml histidinarmem Weichagar für die Plattenbildung auf minimalem Glukoseagar kombiniert. Die Anzahl der Revertanten wurde nach 48-stündiger Inkubation bei 37°C gezählt.

Ergebnis:

Zyklische Dioxadiazocine,die entweder 4-Trifluormethylphenyl (pp'-$CF_3$), 4-Nitrophenyl (pp'-$NO_2$) oder 4-Carboxymethyl esterphenyl (pp'-$CH_3O$ CO) Substituenten aufwiesen, sind bei S. Typhimurium Stämmen mutagen. pp'-$NO_2$ war bei beiden verwendeten Stämmen die aktivste Verbindung.

Das pp'-CF3 Derivat war nur in Gegenwart eines Aktivierungssystems beim Stamm TA100 schwach mutagen. Das pp'-$NO_2$-Derivat ist einerseits direkt mutagen und wird andererseits auch zu einer mutagenen Spezies metabolisiert. Seine direkte Mutagenität war höher in TA98 ,während seine reaktive metabolische Spezies nur in TA100 mutagen oder stärker mutagen war. Eine schwache dosenabhängige Mutagenität in TA100 wurde mit pp'-$CH_3O$ CO in Gegenwart von metabolischem Aktivierungssystem erhalten.

Die direkte Mutagenität von pp'-$NO_2$ kann mit seiner Nitroreduktion in Bakterien in Verbindung gebracht werden.

| Verbindung (µg pro Platte) | Anzahl der Revertanten pro Platte[a)] | | | |
|---|---|---|---|---|
| | TA100 | | TA98 | |
| | +AS[b)] | -AS | +AS | -AS |
| Lösungsmittel (DMSO) | (135) | (102) | (60) | (41) |
| $pp'-CF_3$ 30 | 44 | 8 | 10 | 0 |
| 100 | 86 | 14 | 14 | 0 |
| 300[c)] | 98 | 5 | 24 | 2 |
| 1000[c)] | 96 | 1 | 12 | 0 |
| $pp'-NO_2$ 10 | 22 | 13 | 37 | 65 |
| 30 | 78 | 31 | 88 | 163 |
| 100 | 195 | 100 | 280 | 373 |
| 300[c)] | 357 | 206 | 507 | 839 |
| $pp'-CH_3O\ CO$ 3 | 7 | 6 | 4 | 0 |
| 10 | 18 | 5 | 12 | 0 |
| 30 | 31 | 7 | 19 | 0 |
| 100[c)] | 46 | 6 | 13 | 0 |

Mutagenität von synthetischen zyklischen Dioxadiazocinen in S. Typhimurium Stämmen.

a) Die meisten Zahlen entsprechen Mittelwerten aus zwei Versuchsreihen, die je dreifach gemacht wurden. Die Anzahl der spontanen Revertanten pro Platte (in Gegenwart des Lösungsmittels) wurde abgezogen.

b) AS: die Versuche wurden in Gegenwart (+) oder in Abwesenheit (-) eines metabolischen Aktivierungssystems aus S9 Rattenleber durchgeführt.

c) Die Konzentration bei welcher die untersuchten Verbindungen in S9-Mischung ausfielen.

## Patentansprüche

1. Disubstituierte 3,7-Diaryl-1,5,3,7-dioxodiazocine der allgemeinen Formel

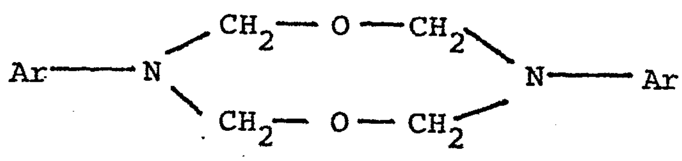

worin Ar eine durch einen elektronenziehenden Rest substituierte Arylgruppe ist.

2. Dioxodiazocine nach Anspruch 1, dadurch gekennzeichnet, daß Ar für eine substituierte Phenylgruppe steht.

3. Dioxodiazocine nach Anspruch 2, dadurch gekennzeichnet, daß die Phenylgruppen in der p-Stellung substituiert sind.

4. Dioxodiazocine nach den Ansprüchen 1 bis 3, dadurch gekennzeichnet, daß die elektronenziehenden Gruppen eine Hammet-Konstante $\sigma$ von 0,20 bis 0,80 besitzen.

5. Dioxodiazocine nach Anspruch 4, dadurch gekennzeichnet, daß die elektronenziehenden Gruppen aus einer gegebenenfalls verzweigten halogenierten Niedrigalkylgruppe mit 1 bis 6 Kohlenstoffatomen, einer halogenierten Cycloalkylgruppe mit bis zu 8 Kohlenstoffatomen, dem Rest $COOR_1$, worin $R_1$ eine Niedrigalkalgruppe mit 1 bis 3 Kohlenstoffatomen bedeutet, oder aus CN oder $NO_2$ besteht.

6. Verfahren zur Herstellung von Dioxodiazocinen gemäß Anspruch 1, dadurch gekennzeichnet, daß mit einer elektronenziehenden Gruppe substituiertes $Ar-NH_2$, vorzugsweise p-substituiertes Anilin, in Chlorwasserstoffsäure mit Nitrit in herkömmlicher Weise diazotiert wird, die erhaltene Diazoniumsalzlösung in eine gekühlte Mischung aus Methylamin und Formaldehyd eingebracht wird, wobei das Verhältnis von Methylamin zu Formaldehyd derart gewählt wird, daß nach der Zugabe der Diazoniumsalzlösung der pH-Wert der Reaktionsmischung zwischen 4 und 5 liegt, anschließend der pH-Wert vorzugsweise auf 1 herabgesetzt wird, die Reaktionsmischung stehengelassen und das ausgefallene Dioxodiazocin isoliert wird.

Europäisches Patentamt

**EUROPÄISCHER RECHERCHENBERICHT**

0166986

Nummer der Anmeldung

EP 85 10 6846

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | CHEMICAL ABSTRACTS, Band 62, Nr. 7, 29. März 1965, Spalten 7753 c - 7754 e, Columbus, Ohio, US; W.V. FARRAR: "Reactions of formaldehyde with aromatic amines" & J. APPL. CHEM. (LONDON) 14(9), 389-99(1964) | 1-6 | C 07 D 273/08 //<br>A 61 K 31/315<br>C 07 C 113/04 |
| X | LIEBIGS ANNALEN DER CHEMIE, Nr. 2, 1981, Seiten 191-197, Weinheim, DE; R. BECKER et al.: "Zur Reaktion von N-Hydroxyharnstoffen mit C1-Bausteinen; ein neuer Zugang zu 2-Alkyl-4-aryl-1,2,4-oxadiazolidin-3-onen" * Insgesamt * | 1-6 | |
| | | | RECHERCHIERTE SACHGEBIETE (Int. Cl.4) |
| | | | C 07 D 273/00 |

Der vorliegende Recherchenbericht wurde für alle Patentansprüche erstellt.

| Recherchenort | Abschlußdatum der Recherche | Prüfer |
|---|---|---|
| DEN HAAG | 02-09-1985 | ALLARD M.S. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument